(19) Europäisches Patentamt
European Patent Office
Office européen des brevets

(11) **EP 4 575 469 A1**

(12) **EUROPEAN PATENT APPLICATION**

(43) Date of publication:
**25.06.2025   Bulletin 2025/26**

(21) Application number: **24214190.1**

(22) Date of filing: **20.11.2024**

(51) International Patent Classification (IPC):
*G01N 21/64* (2006.01)

(52) Cooperative Patent Classification (CPC):
**G01N 21/645; G01N 33/543;** G01N 21/6452;
G01N 2021/7786

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.12.2023   IT 202300027195**

(71) Applicant: **DG GROUP S.p.A.**
**28100 Novara (IT)**

(72) Inventors:
• **RADICE, Dino**
**20010 Marcallo con Casone (MI) (IT)**
• **SILVESTRINI, Lucia**
**20100 Milano (MI) (IT)**

(74) Representative: **Praxi Intellectual Property Milano**
**Via Mario Pagano, 69/A**
**20145 Milano (IT)**

(54) **FLUORESCENCE AMPLIFIER SENSOR**

(57)      A sensor (1) for amplifying fluorescence of a sample comprises:
- a photonic crystal structure (3) of one-dimensional type comprising a first layer (3') in a first material having a first refractive index (n1) and a second layer (3") in a second material having a second refractive index (n2), wherein said first (3") and second (3") layers are repeated alternately to form a plurality of overlapping layers (3', 3'), in an odd number between 7 and 13, such that the outermost layer of the plurality of overlapping layers placed on an upper side of the sensor (1) is a first layer (3') made of the first material, wherein the first refractive index (n1) is greater than the second refractive index (n2) and the ratio of the first refractive index (n1) to the second refractive index (n2) is between 1.5 and 2.2, wherein each layer of the plurality of layers of the photonic crystal structure has a thickness between 9 and 36 nm;
- a free outer surface (4) suitable for supporting said sample, formed on said upper side of the amplifier sensor (1).

FIG. 1

## Description

### Technical field of the invention

The present invention relates, in general, to a sensor capable of amplifying the fluorescence of a sample, and, in particular, to a sensor capable of detecting the presence of target analytes in a sample by fluorescence amplification. In particular, the detection of the target analyte may be carried out by detection of the presence of a fluorophore in the sample, whose fluorescence can be amplified by the sensor according to the present invention.

The term "target analyte" means any chemical and/or biological species whose presence (even trace amounts) in a sample is to be determined.

The present invention, in particular, finds application for the detection of target analytes such as viruses and/or bacteria or their components and/or other substances of medical interest, but it can also be applied for the detection of target analytes of other kinds, as will be described below.

### Prior art

Referring, for example, to the diagnostic field, immunofluorescence techniques are known, which aim to detect in a sample the presence of specific antigens whose counterpart, such as an antibody, is bound to a fluorophore, i.e., a marker that, by absorbing ultraviolet waves (UV, i.e., light radiation having a wavelength between 10 and 400 nm), emits light in the visible spectrum (fluorescence phenomenon).

For example, in the case of so-called direct immunofluorescence, in the search for an antigen in a sample, it is treated with a reagent containing specific antibodies labelled with a fluorophore. If, following exposure to UV light, fluorescence is detected under the microscope, this means that the antigen sought is present in the sample.

Instead, according to the indirect immunofluorescence technique, fluorophore-bound secondary antibodies are used, which in turn are able to bind to primary antibodies specific to the antigen sought.

For effective detection of the target analyte, it is imperative that the instrumentation used for fluorescence detection has high sensitivity and a low limit of detection (LOD, i.e., the lowest analyte concentration that produces a signal). This results in the need to employ complex and expensive instrumentation. In any case, instrumentation employing fluorescence is less sensitive than other available technologies.

Easy-to-use optical amplifier devices, e.g., quartz glass, are also known for UV irradiation, but these allow for low maximum fluorescence values, normally less than 10,000 RFUs (relative fluorescence units).

### Brief summary of the invention

It is an object of the present invention to provide a fluorescence amplifier sensor, capable of achieving higher fluorescence values than those achieved by known optical amplifier devices, which can be employed to make the fluorescence emitted by any sample readily perceptible and which can thus, for example, also be exploited for the detection of the presence of a target analyte in a sample (possibly even in trace amounts) by fluorescence amplification of fluorophore markers.

A further object of the present invention is to provide a fluorescence amplifier sensor that, in addition to the above, is also structurally simple and easy to use.

This and other objects are achieved by a fluorescence amplifier sensor according to claim 1.

The Applicant has verified that such a fluorescence amplifier sensor is capable of achieving fluorescence values significantly higher than the values achievable by optical amplifier devices according to the prior art.

Dependent claims define possible advantageous embodiments of the invention.

### Brief description of the drawings

To better understand the invention and appreciate its advantages, some of its non-limiting exemplary embodiments will be described below, referring to the attached figures, in which:

> figure 1 is a schematic cross-sectional view of a fluorescence amplifier sensor in accordance with a possible embodiment of the invention;
> figure 2 is a schematic sectional view of a one-dimensional photonic crystal;
> figure 3 illustrates a diagram comparing the fluorescence intensity provided by a fluorescence amplifier sensor according to the invention and the one provided by a quartz glass, as a function of the amount of fluorophore-bound antibodies, in a sample solution placed in contact with the sensors;
> figure 4 is a schematic view of a sensor assembly comprising a plurality of fluorescence amplifier sensors according to the present invention, side by side on a support layer.

### Detailed description of the invention

For the purposes of this description and the attached claims, except where otherwise indicated, all numbers expressing amounts, quantities, percentages, and so on, are to be understood as modified in all cases by the term "about." Also, all ranges include any combination of the maximum and minimum points disclosed and include any intermediate ranges within them, which may or may not be specifically listed within.

For the purposes of this description and the attached claims, the words "an" or "one" should be read to include one or at least one and the singular additionally includes the plural unless it is obvious that otherwise is intended.

The present disclosure, in at least one of the aspects mentioned before, may be implemented according to one or more of the following embodiments, optionally combined together.

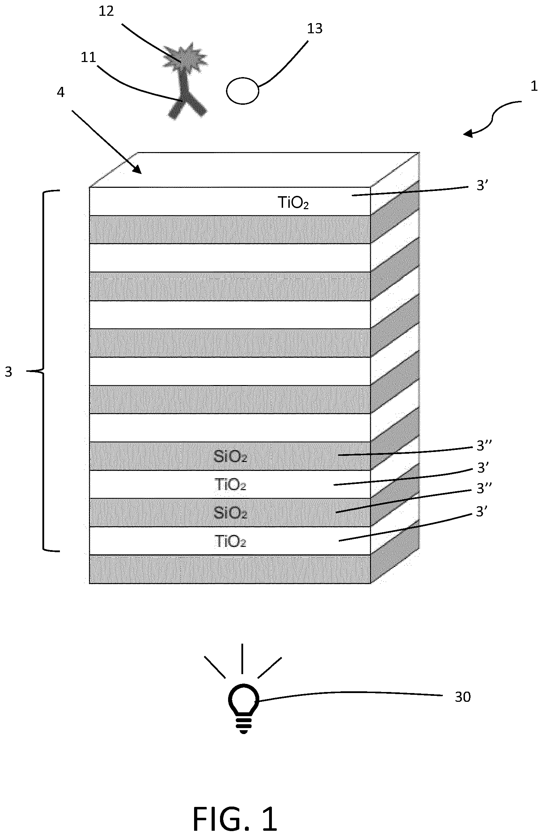

With reference to the attached figure 1, a fluorescence amplifier sensor, in the sense explained in the introductory part of this description, is referred to as a whole as 1. The amplifier sensor 1 can, for example, be made in the form of a label affixed to a utilization instrument (not shown in the figures).

The fluorescence amplifier sensor 1 preferably comprises a support layer 2, having the function of supporting additional overlying layers. The support layer 2 can be made of, but not limited to, polycarbonate, or PVC, or Teslin, or polyester, or PET, or the like. Preferably, support layer 2 is transparent.

Conventionally, the innermost side of the sensor, above which all other layers are placed and where support layer 2 can optionally be provided, is referred to as the "lower side." In contrast, the side of the outermost sensor 1, opposite to the lower side, is conventionally referred to as the "upper side." On the upper side of sensor 1, a free outer surface 4 is provided, i.e., an upper outermost surface with no additional layers resting on it, intended to come into contact with a sample whose fluorescence will be amplified by the sensor 1. The sample can be, for example, either solid or liquid, and in either case it can simply be deposited on the outer surface 4.

The sensor 1 comprises a photonic crystal structure 3 comprising a plurality of layers 3', 3'', ... overlapping each other, and preferably overlapping the support layer 2, where provided. The layers 3', 3'' are also preferably in direct contact with each other. In addition, the photonic crystal structure 3 is preferably in direct contact with the support layer 2.

In optics and microphotonics, a photonic crystal structure is defined as one in which the refractive index has a periodic modulation on scales comparable with the wavelength of light or, more generally, of an electromagnetic radiation. Based on the type of periodic modulation of the refractive index, photonic crystal structures are divided into:

- One-dimensional, having periodicity of refractive index in a single direction (also known as Bragg mirrors);
- Two-dimensional, having refractive index periodicity in two directions;
- Three-dimensional, having refractive index periodicity in three directions.

Advantageously, the photonic crystal structure 3 of sensor 1 is one-dimensional.

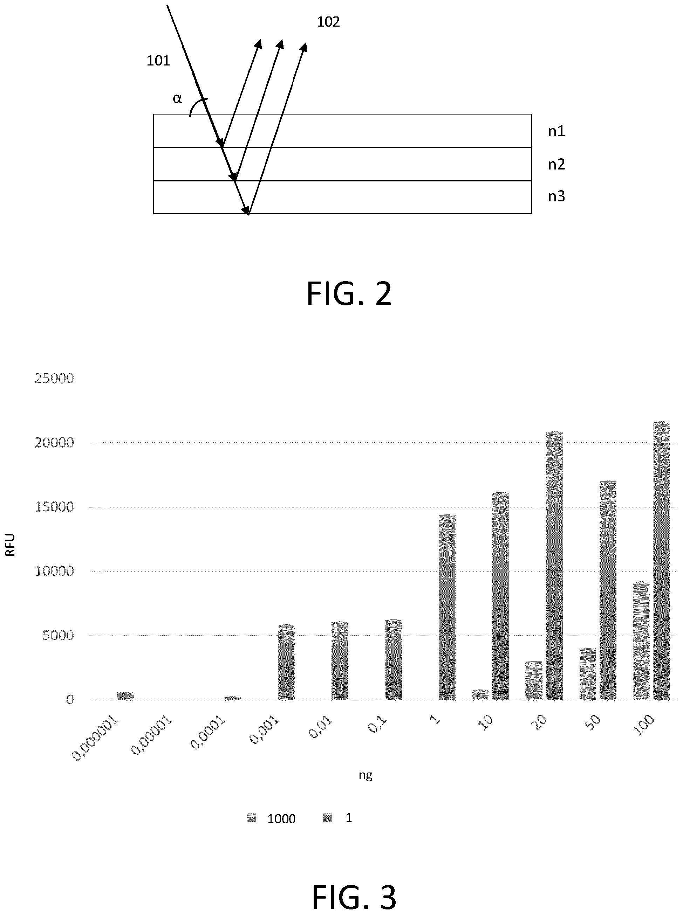

With reference to figure 2, in general, a one-dimensional photonic crystal structure comprises a plurality of layers having respective refractive indices n1, n2, n3. Given a light beam 101 incident at an angle of incidence $\alpha$, the total reflected light is given by a beam 102 of reflected light beams. By appropriately choosing the periodicity and refractive indices of the layers, it is possible to make mirrors that have a very high reflection coefficient in a certain wavelength range. In general, these crystals are designed to manipulate the propagation of light in specific ways, offering precise control over the frequency band of light that can be transmitted or reflected.

The expression "refractive index" (n) of a material means the ratio of the speed of light in vacuum (c) to the speed of light in a medium realized in the material itself (v):

$$n = c/v$$

This (dimensionless) quantity is dependent on the wavelength $\lambda$ of the radiation. For example:

- For titanium dioxide ($TiO_2$) in the rutile form the refractive index n = 2.6142 at a wavelength $\lambda$ = 588 nm;
- For amorphous silicon dioxide ($SiO_2$), the refractive index n = 1.4585 at a wavelength $\lambda$ = 588 nm.

In the following, when referring to refractive indices, the wavelength $\lambda$ = 588 nm will be always taken as a reference.

In accordance with an embodiment, the layers of the photonic crystal structure include a first layer 3' and a second layer 3'' that are repeated alternately from the lower side toward the upper side of sensor 1.

The first 3' and second 3'' layers are made of a first material and a second material having different refractive indices n1, n2, respectively. In particular, the first material has a higher refractive index than the second material:

$$n1 > n2$$

The ratio of the refractive index of the first material of the first layer 3' to that of the second material of the second layer 3'' n1/n2 is comprised between 1.5 and 2.2, preferably between 1.7 and 1.9.

It should be noted that the ratio of refractive indices n1/n2 affects the deflection of light as it passes from one material to another, as illustrated by Snell's law:

$$n1 \cdot \sin(\theta1) = n2 \cdot \sin(\theta2)$$

wherein:

- n1 and n2 are the refractive indices of the two materials,

- θ1 is the angle of incidence with respect to the normal to the surface of the first material, and
- θ2 is the angle of refraction with respect to the normal in the second material.

The ratio of refractive indices n1/n2 thus influences the behaviour of light when it crosses boundaries between different materials. For example, when light passes from a material with a high refractive index to one with a low refractive index, it can be deflected toward the normal, while, in the opposite case, it can be deflected away from the normal. These phenomena underlie various optical effects, such as lens refraction.

Preferably, the refractive index n1 of the first material of the first layer 3' is comprised between 2 and 3.

Preferably, the refractive index n2 of the second material of the second layer 3" is comprised between 1.2 and 1.8, even more preferably between 1.3 and 1.6.

In accordance with an embodiment, the first material is titanium dioxide ($TiO_2$) and the second material is silicon dioxide ($SiO_2$).

The layers of the photonic crystal structure are in an odd number comprised between 7 and 13, preferably 11.

In a photonic crystal structure with an odd number of layers, the arrangement of these layers can be designed to create a photonically active band gap at certain frequencies of light. The photonically active band gap is a region in which the material is designed not to allow certain wavelengths of light to propagate.

According to an embodiment, one or more of the layers of the photonic crystal structure comprises imperfections. These imperfections may include, for example, structural defects, variations in the material composition of the layers, or a break or gap in one or more of the layers, or a non-uniform arrangement of the layers. Imperfections in the layers of a photonic crystal can affect the optical behaviour of the material and can therefore be advantageously exploited. In particular, imperfections can affect the width and position of the band gap, as well as the ability of the photonic crystal structure to guide or confine light, but also to significantly amplify fluorescence.

The layers of the photonic crystal structure 3 are arranged in such a way that the outermost layer, i.e. the layer placed on the upper side of the sensor 1, is a first layer 3' made of the first material, i.e. the material having a higher refractive index. According to an embodiment, such outermost layer is made of titanium dioxide ($TiO_2$).

Each layer of the photonic crystal structure has a thickness comprised between 90 and 360 Å (i.e., between 9 and 36 nm), preferably between 120 and 250 Å (between 12 and 25 nm), preferably between 150 and 200 Å (between 15 and 20 nm), e.g., equal to 180 Å (18 nm).

Advantageously, the photonic crystal structure 3 has a refractive index periodicity in a direction coincident with the direction of development, i.e., overlap, of the layers.

The outer surface 4 of the sensor, which preferably coincides with the free surface of the outermost layer of the photonic crystal structure 3, i.e., on the upper side, which is preferably made of titanium dioxide ($TiO_2$), is intended to support the sample whose fluorescence is to be amplified.

UV irradiation of the fluorescence amplifier sensor 1 is preferably achieved by placing a UV source 30 at the lower side of the sensor, i.e., the side opposite the side on which the sample whose fluorescence is to be amplified is placed. The angle of incidence of the UV light can be 90° (i.e., perpendicular incidence) or it can have a predetermined value different from 90°. It should be observed that, depending on the angle of incidence, a part of the UV light incident on the lower side of the amplifier sensor will be diffused, and this diffusion can be usefully exploited, for example, by placing the amplifier sensor in a chamber such that the UV light is diffused, so that the sensor is irradiated not only on the lower side, but also on the upper side, for example. This diffusion of UV light can enhance fluorescence amplification by the amplifying sensor.

According to an embodiment, the sample is simply applied or placed on the outer surface 4, which therefore requires no special treatment to bind to the sample.

In accordance with a further embodiment, the outer surface 4 is instead bound or is configured to bind to a ligand capable in turn of binding to a molecule, for example a biomolecule, such as a protein (coincident with the target analyte), or even to nucleic acids. Such a ligand may also be bound or capable of binding to a fluorophore, such that a fluorescent compound comprising the ligand, the fluorophore and the target analyte is formed on the outer surface 4. Therefore, when the fluorescent compound is bound to the outer surface 4 of the sensor, upon irradiation with UV light, the fluorophore will become fluorescent and the photonic crystal structure 3 will amplify the light signal emitted by the fluorophore, thereby identifying the presence of the target analyte in the sample.

The fluorescence amplifier sensor according to the invention thus enables the detection, by fluorescence amplification of the fluorophore, of even small amounts of the target analyte in the sample, as well as the detection of larger amounts of the target analyte even with naked eye, without using complex laboratory instrumentation.

It should be noted that the outer surface 4-ligand-fluorophore-target analyte bonds can be made in multiple different ways.

For example, the ligand can be applied to the outer surface 4 during the sensor manufacturing, with or without the bound fluorophore. The fluorophore can be mixed with the sample to bind only to the target analyte, so that when the target analyte binds to the ligand on the outer surface of the sensor, the fluorescence of the fluorophore can be amplified by the sensor.

Alternatively, the fluorophore may be provided already bound to the ligand during the sensor manufacturing. In this case, the fluorophore may have features such that it is fluorescent, and/or emits light within a certain wavelength range (i.e., emits light with a certain colour) only if the ligand has bound to the target analyte.

According to another possible alternative, the ligand and fluorophore are not applied to the outer surface of the sensor during sensor manufacturing, but they are mixed into the sample and bind to the target analyte before the mixture is applied to the outer surface. The latter in that case will be advantageously configured to bind to the ligand (bound to the fluorophore and target analyte) mixed in the sample.

In the above examples, the detection of fluorescence, amplified by sensor 1, is indicative of the presence of the target analyte in the sample. Instead, in the absence of fluorescence (or in the presence of fluorescence with a colour different from the colour sought), the sample does not contain the target analyte.

Examples of possible fluorophores are fluorescein isothiocyanate (FITC), and tetramethylrhodamine isothiocyanate (TRITC).

The configuration of the outer surface 4 may be different depending on the target analyte to be searched for and the ligand that will bind to it.

According to an embodiment, the target analyte is a specific antigen and the outer surface 4 of the sensor is bound to, or is configured to bind to, an antibody suitable for binding to that specific antigen. Such an antibody is, in turn, bound to or capable of binding to a fluorophore, either directly or via a secondary antibody bound to or capable of binding to the fluorophore. Such an embodiment is illustrated schematically in figure 1, in which an antibody 11 capable of binding to a fluorophore 12 and an antigen 13 is shown.

In the case where the ligand is an antibody and the outer surface 4 of the sensor 1 coincides with the outer surface of the outermost layer of the photonic crystal structure, preferably made of Titanium Dioxide ($TIO_2$), the outer surface 4 can be photo-functionalized by irradiation with an ultraviolet UV light. The ultraviolet light can have a wavelength comprised between 300 and 400 nm. The surface can be irradiated with an intensity between 5 and 30 mW/cm$^2$, preferably for between 0.1 and 2 hours. Such treatment enables electrostatic immobilization, without the use of chemical reagents, of antibodies on the outer surface 4, as demonstrated, for example, in Huan Deng, Xiangqin Liu, Jie Chen, Yi He, Lanke Lin, Xin Liu, Jiang Chen and Xiaoqi Liu, "Photo-functionalized TiO2 film for facile immobilization of EpCAM antibodies and efficient enrichment of circulating tumor cells," Front. Pharmacol., Feb. 28, 2023, Sec. Pharmacology of AntiCancer Drugs, Volume 14 - 2023, DOI 10.3389/fphar.2023.1126602, the content of which is fully incorporated herein by reference.

Figure 3 shows a comparison between the fluorescent signal emitted by an amplifier sensor 1 in accordance with the present invention and a quartz glass 1000 for different amounts of fluorophore-bound antibodies in a sample solution placed in contact with the sensors (amounts in ng). In the example, a FITC-conjugated fluorescent antibody was specifically considered. As can be seen, the relative fluorescence intensity (RFU) of the sensor according to the invention is strongly amplified for any amount of antibody (up to more than 20,000 RFU) and is also intense already at low amounts (of the order of 1 pg), compared to the quartz glass.

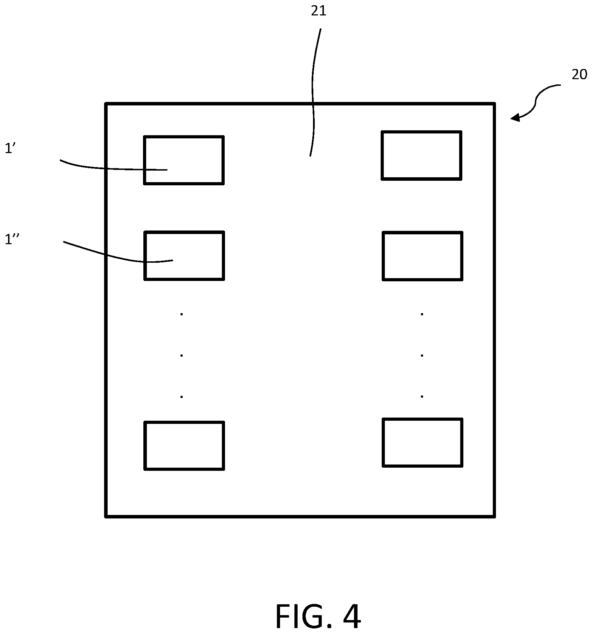

According to a further aspect of the present invention, it is possible to provide an amplifier sensor assembly 20 comprising a plurality of amplifier sensors according to the present invention 1', 1", placed side by side on a support layer 21, each suitable for identifying, in the manner previously described, different target analytes (figure 4). In other words, the amplifier sensor assembly 20 comprises an array of amplifier sensors, each made in such a way as to be selective toward a specific target analyte. In this way, sensor assembly 20 can selectively detect multiple target analytes simultaneously by each of the sensors in the array, even from the same sample.

The fluorescence amplifier sensor according to the present invention can find application in a variety of fields, of which some non-limiting examples are given below:

- Diagnostics in medical or veterinary settings:

    ○ diagnosis, by demonstration of the pathogen or its components, of bacterial (such as: Lyme disease, Brucellosis, Syphilis), viral (such as: HIV, Hepatitis B,C) and fungal (such as pathogenic yeast infections) infections;

    ○ detection of viruses (such as: Coronavirus, HIV, Hepatitis, Ebola, Norovirus, Influenza, West Nile, Zika);

    ○ diagnosis, by demonstration of specific clinically validated markers, of autoimmune diseases, due to abnormal production by the immune system of autoantibodies (for example: autoantibodies against insulin-producing cells in the pancreas in type I diabetes);

    ○ detection and quantification of hormones (e.g., human chorionic gonadotropin (hCG), follicle-stimulating hormone (FSH), testosterone);

    ○ Screening of donated blood and blood products (e.g., for detection of viral infections, such as HCV and HIV);

    ○ Detection of drugs (for example: amphetamines, cocaine and metabolites of those substances);

    ○ detection of tumoral markers (for example: prostate specific antigen (PSA) for prostate cancer diagnosis);

    ○ veterinary diagnostics, for example: detection of the causative agent of African swine fever, avian influenza virus, bovine parvovirus, canine adenovirus, coronavirus, equine infectious anemia, feline leukemia, etc.);
◦ Blood allergy testing by measuring IgE isotype antibodies directed against specific allergens;
◦ Analysis of nucleic acids;

- Non-diagnostic applications:

◦ Detection of transcription factor proteins for example in agronomy;
◦ Improvements in optical devices, such as optical memories;
◦ Use in laboratory instrumentation;
◦ Fundamental research based on spectroscopic techniques (e.g., in life sciences);
◦ Materials chemistry;
◦ Internet of Things (IoT);
◦ Biosafety:

■ Surveillance of epidemics;
■ Biological weapons/bioterrorism testing;
■ Testing in cosmetics (for example: testing for metal contamination);

◦ Detection of contaminants:

■ Detection and screening of toxins and/or contaminating pathogens in food (e.g.: *Salmonella sp., Escherichia coli, Campylobacter sp., Staphylococcus aureus*);
■ Detection and screening of environmental pollutants (e.g., detection of heavy metals in water or soil);
■ Pesticide detection;
■ Gunpowder detection.

To the above description of the fluorescence amplifier sensor the skilled person, in order to meet specific contingent needs, may make numerous additions, modifications, or substitutions of elements with functionally equivalent ones, without, however, departing from the scope of the attached claims.

**Claims**

1. Sensor (1) for amplifying fluorescence of a sample, comprising:

- a photonic crystal structure (3) of one-dimensional type comprising a first layer (3') in a first material having a first refractive index (n1) and a second layer (3") in a second material having a second refractive index (n2), wherein said first (3") and second (3") layers are repeated alternately to form a plurality of overlapping layers (3', 3'), in an odd number between 7 and 13, such that the outermost layer of the plurality of overlapping layers placed on an upper side of the sensor (1) is a first layer (3') made of the first material, wherein the first refractive index (n1) is greater than the second refractive index (n2) and the ratio of the first refractive index (n1) to the second refractive index (n2) is between 1.5 and 2.2, wherein each layer of the plurality of layers of the photonic crystal structure has a thickness between 9 and 36 nm;
- a free outer surface (4) suitable for supporting said sample, formed on said upper side of the amplifier sensor (1).

2. Amplifier sensor (1) according to claim 1, wherein said outer surface (4) is formed on the outermost layer of the plurality of overlapping layers of the photonic crystal structure (3) made in the first material.

3. Amplifier sensor (1) according to claim 1 or 2, wherein the ratio of the first refractive index (n1) to the second refractive index (n2) is between 1.7 and 1.9.

4. Amplifier sensor (1) according to any one of the preceding claims, wherein the first refractive index (n1) is between 2 and 3.

5. Amplifier sensor (1) according to any one of the preceding claims, wherein the second refractive index (n2) is between 1.2 and 1.8.

6. Amplifier sensor (1) according to any one of the preceding claims, wherein the first material of the first layer (3') is titanium dioxide ($TiO_2$) and/or the second material of the second layer is silicon dioxide ($SiO_2$).

7. Amplifier sensor (1) according to any one of the preceding claims, wherein at least one of the layers of the photonic crystal structure comprises imperfections.

8. Amplifier sensor (1) according to any one of the preceding claims, wherein the outer surface (4) of the sensor is bonded to or is configured to bind to a ligand capable of binding to a target analyte contained in the sample and bonded to or capable of binding to a fluorophore.

9. Amplifier sensor (1) according to the preceding claim, wherein the target analyte contained in the sample is a specific antigen and the outer surface (4) of the sensor is bonded to or is configured to bind to an antibody suitable for binding to said specific antigen.

10. Amplifier sensor (1) according to the preceding claim, wherein said outer surface (4) is formed on said outermost layer of the plurality of overlapping layers of the photonic crystal structure made of titanium dioxide ($TiO_2$), wherein said outer surface (4) is photo-functionalised by irradiation with an ultraviolet light having a wavelength between 300 and 400 nm.

11. Amplifier sensor (1) according to the preceding claim, wherein the outer surface (4) is photo-functionalised with said ultraviolet light with an intensity between 5 and 30 mW/cm$^2$ for a time between 0.1 and 2 hours.

12. Amplifier sensor assembly (20) comprising a support layer (21) and a plurality of amplifier sensors (201', 201") according to any one of the preceding claims placed side by side on said support layer (21), each suitable for identifying different target analytes in said sample.

FIG. 1

FIG. 2

FIG. 3

FIG. 4

Europäisches
Patentamt

European
Patent Office

Office européen
des brevets

# EUROPEAN SEARCH REPORT

**Application Number**

EP 24 21 4190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2015/338402 A1 (LAKOWICZ JOSEPH R [US] ET AL) 26 November 2015 (2015-11-26) * paragraphs [0051], [0081], [0096], [0114], [0122], [0126] * * figures 4B,7 * | 1-12 | INV. G01N21/64 |
| X | INOUYE H ET AL: "OPTICAL PROPERTIES OF A TOTAL-REFLECTION-TYPE ONE-DIMENSIONAL PHOTONIC CRYSTAL", IEEE JOURNAL OF QUANTUM ELECTRONICS, IEEE, USA, vol. 7, no. 38, 1 July 2002 (2002-07-01), pages 867-871, XP001141667, ISSN: 0018-9197, DOI: 10.1109/JQE.2002.1017599 | 1,2,6 | |
| Y | * abstract * * figures 1,4 * * page 867, right-hand column, last paragraph - page 868, left-hand column * * page 869, left-hand column * | 1-12 | |
| Y | ZHANG YIFAN ET AL: "Simultaneous Trapping and Manipulation of Micro-Specimens Using an All-Dielectric One-Dimensional Photonic Crystal", JOURNAL OF LIGHTWAVE TECHNOLOGY, IEEE, USA, vol. 41, no. 5, 16 January 2023 (2023-01-16), pages 1511-1518, XP011935478, ISSN: 0733-8724, DOI: 10.1109/JLT.2023.3237611 [retrieved on 2023-01-17] * page 1511, right-hand column * * page 1516, left-hand column * * figure 1 * | 1-12 | TECHNICAL FIELDS SEARCHED (IPC) G01N |

-/--

The present search report has been drawn up for all claims

| Place of search | Date of completion of the search | Examiner |
|---|---|---|
| Munich | 1 April 2025 | D'Alessandro, Davide |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 24 21 4190

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Michelotti Francesco: "SENSITIVE LABEL-FREE AND FLUORESCENCE CANCER BIOMARKER DETECTION USING ONE DIMENSIONAL PHOTONIC CRYSTAL BIOCHIPS", , 1 December 2016 (2016-12-01), XP093183374, Retrieved from the Internet: URL:https://iris.uniroma1.it/retrieve/e383 5316-51b3-15e8-e053-a505fe0a3de9/Tesi%20do ttorato%20Sinibaldi * page 28 * * figure 2.7 * | 1-12 | |
| A | BADUGU RAMACHANDRAM ET AL: "Radiative decay engineering 6: Fluorescence on one-dimensional photonic crystals", ANALYTICAL BIOCHEMISTRY, ACADEMIC PRESS, AMSTERDAM, NL, vol. 442, no. 1, 27 July 2013 (2013-07-27) , pages 83-96, XP028717306, ISSN: 0003-2697, DOI: 10.1016/J.AB.2013.07.021 * abstract * * page 84, right-hand column * * figure 3 * | 1-12 | |
| Y,D | DENG HUAN ET AL: "Photo-functionalized TiO2 film for facile immobilization of EpCAM antibodies and efficient enrichment of circulating tumor cells", FRONTIERS IN PHARMACOLOGY, vol. 14, 28 February 2023 (2023-02-28), XP093183548, CH ISSN: 1663-9812, DOI: 10.3389/fphar.2023.1126602 * abstract * * page 3, left-hand column * | 10,11 | TECHNICAL FIELDS SEARCHED (IPC) |

The present search report has been drawn up for all claims

| Place of search | Date of completion of the search | Examiner |
|---|---|---|
| Munich | 1 April 2025 | D'Alessandro, Davide |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 4190

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2025

| Patent document cited in search report | Publication date | Patent family member(s) | Publication date |
|---|---|---|---|
| US 2015338402 A1 | 26-11-2015 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HUAN DENG** ; **XIANGQIN LIU** ; **JIE CHEN** ; **YI HE** ; **LANKE LIN** ; **XIN LIU** ; **JIANG CHEN** ; **XIAOQI LIU**. Photo-functionalized TiO2 film for facile immobilization of EpCAM antibodies and efficient enrichment of circulating tumor cells. *Front. Pharmacol.*, 28 February 2023, vol. 14